# EUROPEAN PATENT APPLICATION

(11) **EP 2 826 428 A1**
(43) Date of publication of application: **21.01.2015**
(21) Application number: 14188172.2
(22) Date of filing: 01.10.2013
(51) Int. Cl.: A61B 17/29, A61B 1/12, A61B 19/00, A61B 1/313, A61B 17/00, A61B 17/32, A61B 17/34

(54) **Laparoscopic lens wipe feature on surgical device shaft or jaw member**

(30) Priority: 02.10.2012 US 201261708677 P; 11.09.2013 US 201314023500
(62) Divisional of application: 13186950.5
(71) Applicant: Covidien LP, Mansfield, MA 02048 (US)
(72) Inventor: Schaning, Matthew, Cambridge, Wisconsin 53523 (US)
(74) Representative: Tanner, James Percival

(57) **Abstract**

A first surgical instrument comprising a handle assembly defining a proximal end of the first surgical instrument, an extension member extending distally from the handle assembly and defining a distal end of the first surgical instrument, the extension member defining an external surface, and the handle assembly configured to enable a user to move the extension member, a cleaning material disposed on the external surface of the extension member at the distal end thereof, the cleaning material configured to clean at least a portion of a second surgical instrument in proximity to the first surgical instrument, the first surgical instrument and the second surgical instrument defining a distance therebetween, the cleaning material configured to clean at least a portion of the second surgical instrument when the distance between the first surgical instrument and the second surgical instrument is reduced, the cleaning material including a blade-like structure formed from a flexible material, characterized in that the flexible material is made from one of cloth, plastic, rubber or silicone.

## Description

### CROSS-REFERENCE TO RELATED APPLICATIONS

This application claims the benefit of and priority to U.S. Provisional Patent Application No. 61/708,677, filed October 2, 2012, the entire disclosure of which is incorporated by reference herein

### BACKGROUND

### Technical Field

This application relates to maintaining optical clarity of a surgical instrument.

### Description of Related Art

Known practices of cleaning a lens of a laparoscopic instrument scope involve removing the scope from the patient, wiping with a cloth, reinserting the scope, and then finding the proper location in the body again. This can occur multiple times during a procedure and increases overall procedure time as fog and/or smoke and/or bodily fluids often accumulate on the laparoscopic lens during a laparoscopic surgical procedure. Moreover, removing a device and reinserting increases infection risk, albeit a very small risk as this is performed in a sterile field.. Surgeons typically must remove the laparoscope from the patient and lose time re-orienting themselves and their instruments to the operative site.

### SUMMARY

To advance the state of the art with respect to maintaining optical clarity of a surgical instrument during a surgical procedure, the present disclosure relates to a first surgical instrument having a cleaning material configured to clean a second surgical instrument. More particularly, in one embodiment, the first surgical instrument includes a handle assembly defining a proximal end of the first surgical instrument and an extension member extending distally from the handle assembly and defining a distal end of the first surgical instrument. The extension member defines an external surface. The handle assembly is configured to enable a user to move the extension member. The improvement includes a cleaning material disposed on the external surface of the extension member at the distal end. The cleaning material is configured to clean at least a portion of a second surgical instrument in proximity to the first surgical instrument. The first surgical instrument and the second surgical instrument define a distance therebetween. The cleaning material is configured to clean at least a portion of the second surgical instrument when the distance between the first surgical instrument and the second surgical instrument is reduced.

In one embodiment, the extension member is a shaft having a pair of jaw members at a distal end of the shaft. The jaw members are movable about a pivot from a first position wherein the jaw members are disposed in spaced relation relative to one another to a at least a second position wherein the jaw members are closer to one another for grasping tissue. The first surgical instrument is configured to enable a user to move the shaft and the pair of jaw members. The shaft and the jaw members each define external surfaces. The first surgical instrument includes a cleaning material disposed on the external surface of at least one jaw member or disposed on the external surface of the shaft.

The cleaning material may be disposed on the distal end of the shaft in proximity to the pair of jaw members. The second surgical instrument may be a laparoscopic instrument including a lens surface and the cleaning material is configured to clean at least a portion of the lens of the laparoscopic instrument when the distance between the first surgical instrument and the second surgical instrument is reduced.

In one embodiment, the cleaning material may be adhered to the extension member, e.g., at least one jaw member or the shaft, via an adhesive adhering a surface of the cleaning material to the external surface of the extension member, e.g., to the external surface of the at least one jaw member or to the external surface of the shaft respectively.

In one embodiment, the extension member, such as the shaft, defines a depression therein and the cleaning material is disposed in the depression in the extension member.

In yet another embodiment, the cleaning material includes a blade-like structure. The blade-like structure may be configured to clean at least a portion of the second surgical instrument when the distance between the first surgical instrument and the second surgical instrument is reduced. The blade-like structure may be formed from a flexible material. The flexible material may be made from one of cloth, rubber or silicone. In one embodiment, when the second surgical instrument is a laparoscopic instrument including a lens surface, the blade-like structure may be configured to clean at least a portion of the lens of the laparoscopic instrument when the distance between the first surgical instrument and the second surgical instrument is reduced.

In one embodiment, the extension member defines a longitudinal axis and the first surgical instrument may be configured to reciprocally move in the direction of the longitudinal axis to enable the cleaning material to clean the second surgical instrument when the distance between the first surgical instrument and the second surgical instrument is reduced.

In still another embodiment, the first surgical instrument may be configured to enable the cleaning material to move in a curved path to clean the second surgical instrument when the distance between the first surgical instrument and the second surgical instrument is reduced.

In yet another embodiment, the extension member defines a longitudinal axis and the first surgical instrument may be configured to rotate the cleaning material around the longitudinal axis to enable the cleaning material to clean the second surgical instrument when the distance between the first surgical instrument and the second surgical instrument is reduced.

The present disclosure relates also to a method of cleaning at least a portion of a second surgical instrument in proximity to a first surgical instrument. In one embodiment, the first surgical instrument includes a handle assembly and an extension member that extends distally from the handle assembly. The method includes disposing a cleaning material on an external surface of the extension member; positioning the first surgical instrument in proximity to a second surgical instrument to define a distance between the first and second surgical instruments; reducing the distance between the first surgical instrument and the second surgical instrument; and cleaning at least a portion of the second surgical instrument with the cleaning material when the distance between the first surgical instrument and the second surgical instrument is reduced.

In one embodiment, the extension member is a shaft having a pair of jaw members at a distal end thereof. The jaw members are movable about a pivot from a first position wherein the jaw members are disposed in spaced relation relative to one another to at least a second position wherein the jaw members are closer to one another for grasping tissue. The step of disposing the cleaning material includes disposing the cleaning material on an external surface of the shaft or an external surface of at least one jaw member. The step of disposing the cleaning material includes disposing the cleaning material on the external surface of the shaft at the distal end of the shaft in proximity to the pair of jaw members.

In one embodiment, the second surgical instrument is a laparoscopic instrument including a lens surface and the method includes cleaning at least a portion of the lens of the laparoscopic instrument with the cleaning material when the distance between the first surgical instrument and the second surgical instrument is reduced.

In one embodiment, the step of disposing the cleaning material may include adhering the cleaning material to the external surface of the extension member, e.g., to the external surface of at least one jaw member or to the external surface of the shaft, via an adhesive adhering a surface of the cleaning material to the external surface of the extension member.

In one embodiment, the extension member defines a depression therein and the step of disposing the cleaning material may include disposing the cleaning material in the depression in the extension member.

In yet another embodiment, the step of disposing the cleaning material on an external surface of the extension member includes disposing a cleaning material having a blade-like structure, and the step of cleaning at least a portion of the second surgical instrument with the cleaning material when the distance between the first surgical instrument and the second surgical instrument is reduced may include cleaning at least a portion of the second surgical instrument with the cleaning material having a blade-like structure.

In one embodiment, the second surgical instrument is a laparoscopic instrument including a lens surface, and the method includes cleaning at least a portion of the lens of the laparoscopic instrument with the cleaning material having a blade-like structure.

In yet another embodiment, the extension member defines a longitudinal axis, and the method includes the step of reciprocally moving the first surgical instrument in the direction of the longitudinal axis such that the cleaning material cleans the second surgical instrument when the distance between the first surgical instrument and the second surgical instrument is reduced.

In one embodiment, the step of cleaning may include moving the cleaning material in a curved path to clean the second surgical instrument when the distance between the first surgical instrument and the second surgical instrument is reduced.

In yet another embodiment, the extension member defines a longitudinal axis and the step of cleaning includes rotating the cleaning material around the longitudinal axis such that the cleaning material cleans the second surgical instrument when the distance between the first surgical instrument and the second surgical instrument is reduced.

### BRIEF DESCRIPTION OF THE DRAWINGS

Various embodiments will be described herein below with reference to the drawings wherein:
FIG. 1 is a side view of surgical instrument having a cleaning material disposed on a shaft of the instrument according to one embodiment of the present disclosure;
FIG. 1A is a detailed view of the cleaning material of FIG. 1 disposed on the shaft;
FIG. 2 is a cross-sectional view of the abdominal cavity of a patient in which the surgical instrument of FIG. 1 having a cleaning material disposed on a shaft of the instrument is inserted into the abdominal cavity in proximity to a second surgical instrument having a lens which can be cleaned by the cleaning material;
FIG. 3 is cross-sectional view of the abdominal cavity of FIG. 2 in which the cleaning material is in position to directly contact and clean the lens of the second surgical instrument;
FIG. 4 is a perspective view the shaft of the surgical instrument of FIG. 1 wherein the shaft defines a depression therein and wherein the cleaning material is disposed in the depression in the shaft according to one embodiment of the present disclosure;
FIG. 5 is a perspective view of the shaft of the surgical instrument of FIG. 1 wherein the cleaning material has a blade-like configuration according to one embodiment of the present disclosure; and
FIG. 6 is a perspective view of jaw members of the surgical instrument of FIG. 1 surgical instrument having a cleaning material disposed on a jaw member of the instrument.

### DETAILED DESCRIPTION

The embodiments of the present disclosure relate to a surgical instrument having a cleaning material that is disposed on a shaft or on a jaw member of a first surgical instrument in which the cleaning material is configured to clean at least a portion of at least a second surgical instrument in proximity to the first surgical instrument. The embodiments of the present disclosure advance the state of the art of surgical procedures by speeding procedure time, by requiring fewer steps for cleaning, and by lowering the risk of infection.

The surgical instrument having a cleaning material according to the embodiments of the present disclosure eliminates time costly steps by incorporating a laparoscopic wipe feature on the shaft of a commonly used laparoscopic surgical instrument such as a radio frequency (RF) sealer/divider, manufactured by Covidien, Inc (Boulder, Colorado, USA) under the trademark LIGASURE™ or an ultrasonic dissector, manufactured by Covidien, Inc. (Boulder, Colorado, USA) under the trademark SONICISION™.

The cleaning material or wipe may be made from a material that is felt-like and adhered to the shaft of the device near the jaws. Other material options include rubbers/silicones that behave similarly to windshield wipers.

It is worthy to note that any reference in the specification to "one embodiment" or "an embodiment" means that a particular feature, structure, or characteristic described in connection with the embodiment is included in at least one embodiment. The appearances of the phrase "in one embodiment" in various places in the specification are not necessarily all referring to the same embodiment.

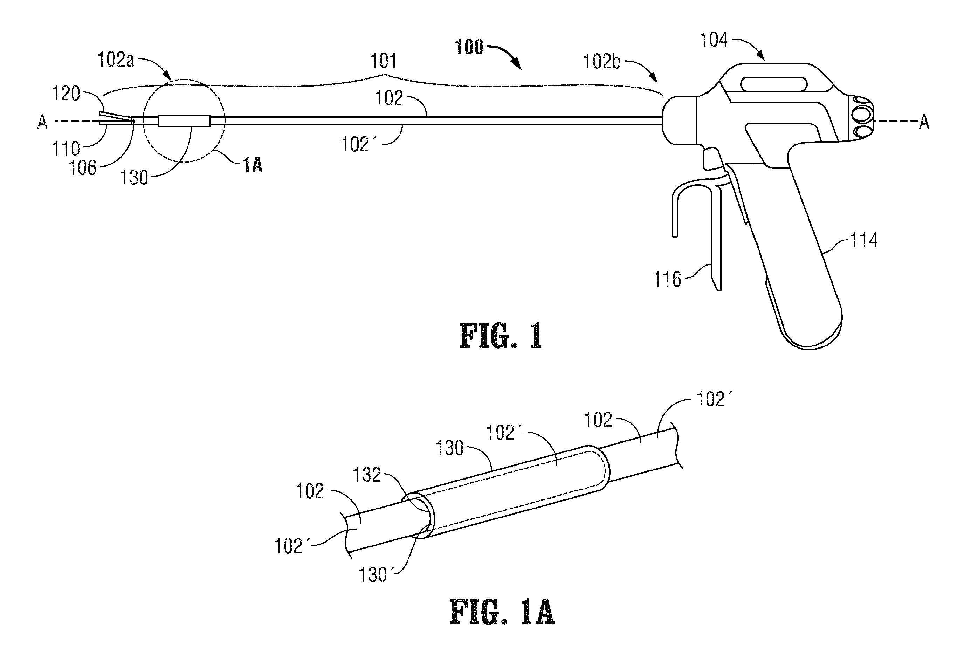

Turning now to FIG. 1, there is disclosed an exemplary first surgical instrument 100 that includes a handle assembly 104. An extension member 101 extends distally from the handle assembly 104 to define a proximal end 102b and a distal end 102a of the first surgical instrument 100. The extension member 101 defines an external surface 102'. In the exemplary embodiment of FIG. 1, the handle assembly 104 may include a handle 114 that includes a movable trigger or actuator 116 on a distal side of the handle 114.

More particularly, as shown in the exemplary embodiment of FIG. 1, the first surgical instrument is an electrosurgical forceps wherein the extension member 101 includes a shaft 102 having a pair of jaw members, e.g., lower jaw member 110 and upper jaw member 120, at distal end 120a of the shaft 102. The jaw members 110 and 120 are movable about a pivot 106 from a first position wherein the jaw members 110 and 120 are disposed in spaced relation relative to one another, as shown in FIG. 1, to a second position wherein the jaw members 110 and 120 are closer to one another (not shown) for grasping tissue (not shown). In the example herein of an electrosurgical forceps, at least one jaw member 110 and/or 120 is adapted to connect to a source of electrical energy (not shown) and such that the jaw members 110 and 120 are capable of conducting energy to tissue held between the jaw members 110 and 120. The movable trigger or actuator 116 actuates a drive assembly, included within shaft 102, to move the jaw members 110 and 120 relative to one another.

As can be appreciated from the foregoing, the first surgical instrument 100 is configured to enable a user, e.g., a surgeon or a remotely controlled machine (neither of which are shown), to move the shaft 102 and the pair of jaw members 110 and 120.

As illustrated in FIG. 1A, in one embodiment, a cleaning material 130 may be adhered to the shaft 102 via an adhesive 132 adhering a surface 130' of the cleaning material 130 to external surface 102' of the extension member 101 as represented by the shaft 102. 11. The cleaning material may be made from cloth, rubber or silicone or other suitable material.

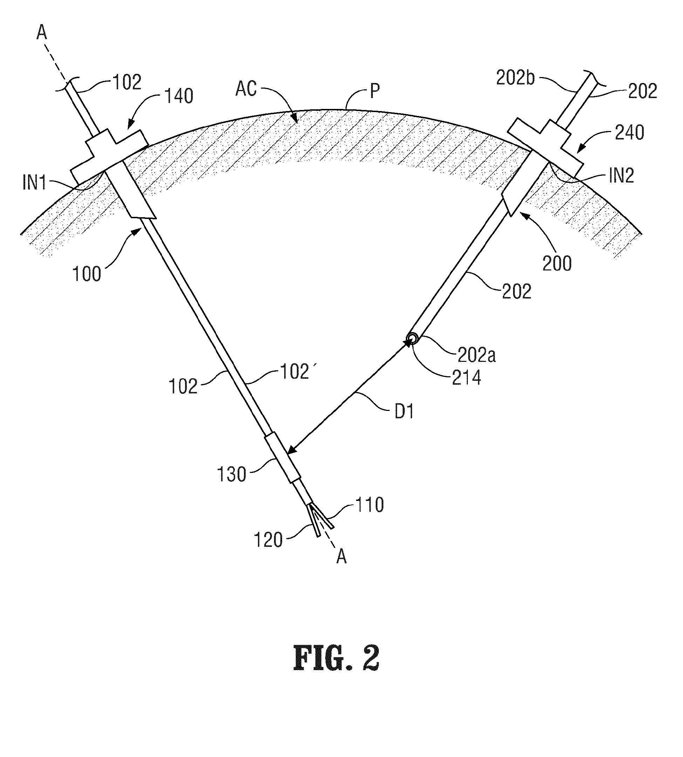

In conjunction with FIG. 2, the cleaning material 130 is disposed on the shaft 102 in proximity to the distal end 102a. The shaft 102 and the jaw members 110 and 120 of the first surgical instrument 100 are disposed through a first trocar 140 that penetrates a first incision **IN1** into the abdominal cavity **AC** in a patient **P.**

At least a second exemplary surgical instrument 200 includes a shaft 202 having a distal end 202a and a proximal end 202b. The distal end 202a of the shaft 202 is disposed through a second trocar 240 that penetrates a second incision **IN2** into the abdominal cavity **AC** of the patient **P.** The second surgical instrument 200 may be inserted through the second trocar 240 via a movable handle (not shown) that may be disposed in the vicinity of the proximal end 202b of the shaft 202. The first surgical instrument 100 and the second surgical instrument 200 are positioned through the first and second incisions **IN1** and **IN2** at an angle with respect to each other to define a variable distance **D1** between the distal end 102a of the first surgical instrument 100 and the distal end 202a of the second surgical instrument 200.

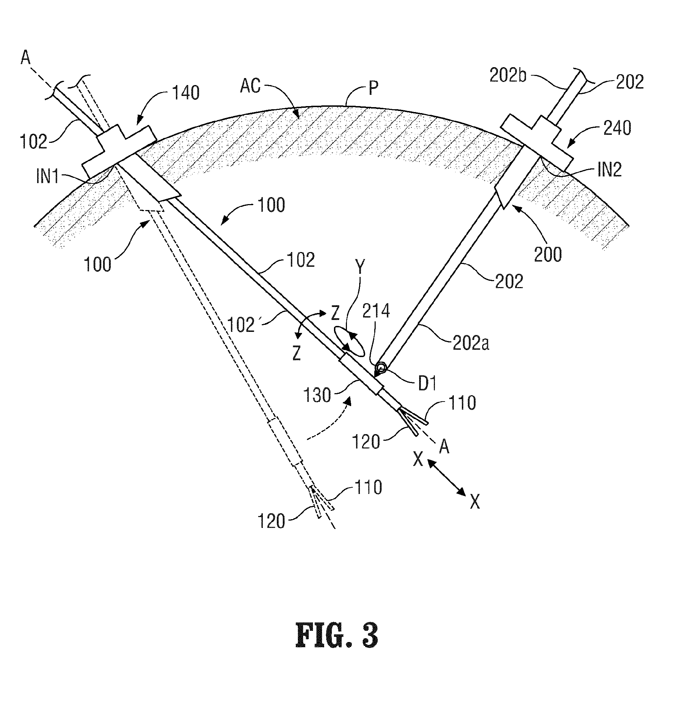

Turning now to FIG. 3, when the variable distance **D1** is reduced sufficiently, such as by moving either handle 104 of the first surgical instrument 100 or the movable handle of the second surgical instrument 200 or both, the cleaning material 130 is moved into position in proximity to the distal end 202a of the second surgical instrument 200 to contact the second surgical instrument 200 such that the cleaning material 130 is thus configured to clean at least a portion of the second surgical instrument 200.

As illustrated in FIGS. 2 and 3, the exemplary second surgical instrument 200 is a laparoscopic instrument that includes a lens surface 214 disposed at the distal end 202a of the shaft 202. As illustrated in FIGS. 1, 2 and 3, the extension member 101, as represented by shaft 102, defines a longitudinal axis **A-A** such that the shaft 102 can move in multiple directions to enable the cleaning material 130 to clean the second surgical instrument 200, and more particularly in the exemplary embodiment of FIGS. 2 and 3, the lens 214 of the second or laparoscopic instrument 200.

In one embodiment, the first surgical instrument 100 can be reciprocally moved, and thus is configured to reciprocally move, in the direction of the longitudinal axis **A-A** as shown by the double-headed arrow **X** to enable the cleaning material 130 to clean the second surgical instrument 200 when the distance **D1** is reduced sufficiently as described above.

In one embodiment, the first surgical instrument 100 can be moved in a curved path, and thus is configured to move in a curved path, as shown by the curved path **Y** to enable the cleaning material 130 to move in the curved path **Y** to clean the second surgical instrument 200 when the distance **D1** is reduced sufficiently again as described above.

The first surgical instrument 100 can also be caused to rotate the cleaning material 130 around the longitudinal axis **A-A,** and thus the first surgical instrument 100 is configured to rotate the cleaning material 130 around the longitudinal axis **A-A,** as shown by the double-headed circular arrow **Z** to enable the cleaning material 130 to clean the second surgical instrument 200 when the distance **D1** is reduced sufficiently as described above The rotation of the cleaning material 130 may be effected by rotation of the shaft 102 around the longitudinal axis **A-A.**

As can be appreciated by those skilled in the art, by means of the aforementioned means of motion of the cleaning material 130, the cleaning material 130 is thus also configured to clean at least a portion of the lens 214 of the second or laparoscopic instrument 200 when the distance **D1** between the first surgical instrument 100 and the second surgical instrument 200 is reduced sufficiently as described above.

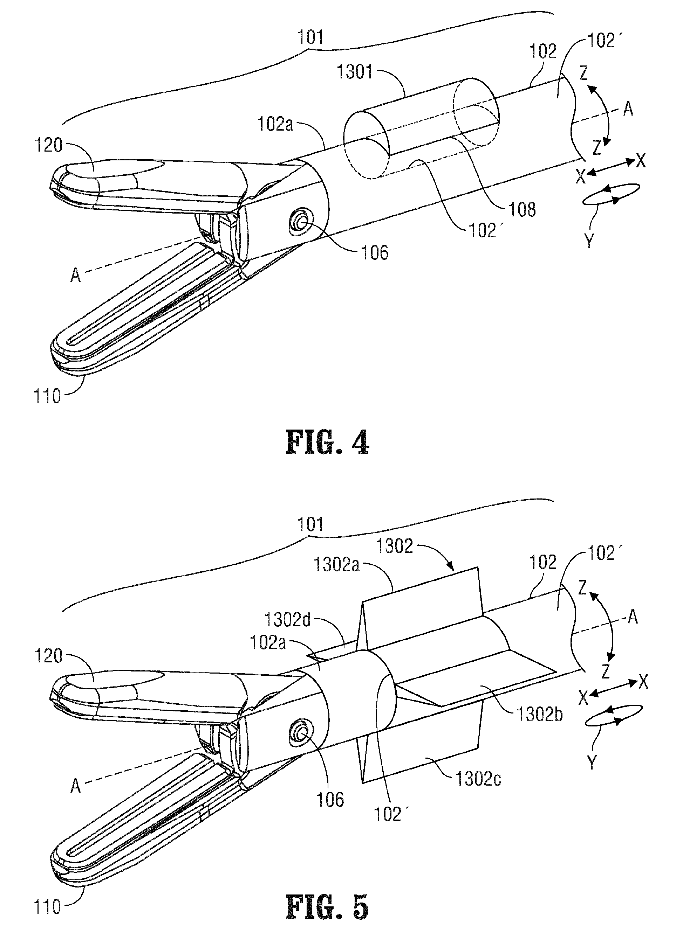

FIG. 4 illustrates one embodiment of the present disclosure wherein the extension member 101 represented by the shaft 102 defines a depression 108 in external surface 102' in proximity to distal end 102a and a cleaning material 1301 is disposed in the depression 108 in the shaft 102 to enable more secure retention of the cleaning material 1301 within the depression 108 and attachment to the shaft 102 during a surgical procedure. As before, the cleaning material 1301 may be formed from a flexible material such as cloth, rubber or silicone.

Although the cleaning material 1301 may be moved in the **X** and/or **Y** directions described above with respect to FIG. 3, the cleaning material 1301 is particularly suitable for rotational motion in the **Z** direction as also described above with respect to FIG. 3. Thus, the cleaning material 1301 is configured to clean at least a portion of the second surgical instrument 200 when the distance **D1** between the first surgical instrument 100 and the second surgical instrument 200 is reduced sufficiently as described above.

FIG. 5 illustrates one embodiment of the present disclosure wherein a cleaning material 1302, disposed on external surface 102' of the extension member 101 represented by shaft 102, is defined by a blade-like structure that includes, for example, first, second, third and fourth blade-like projections 1302a, 1302b, 1302c and 1302d that radially project outwardly from the shaft centerline axis **A-A.** The blade-like structure 1302 may be formed from a flexible material such as cloth, rubber or silicone.

In a similar manner, although the blade like projections 1302a to 1302d may be moved in the **X** and/or **Y** directions described above with respect to FIG. 3, the blade-like projections 1302a to 1302d are also particularly suitable for rotational motion in the **Z** direction as described above with respect to FIG. 3. Thus, the blade-like structure of cleaning material 1302 is configured to clean at least a portion of the second surgical instrument 200 when the distance **D1** between the first surgical instrument 100 and the second surgical instrument 200 is reduced sufficiently as described above.

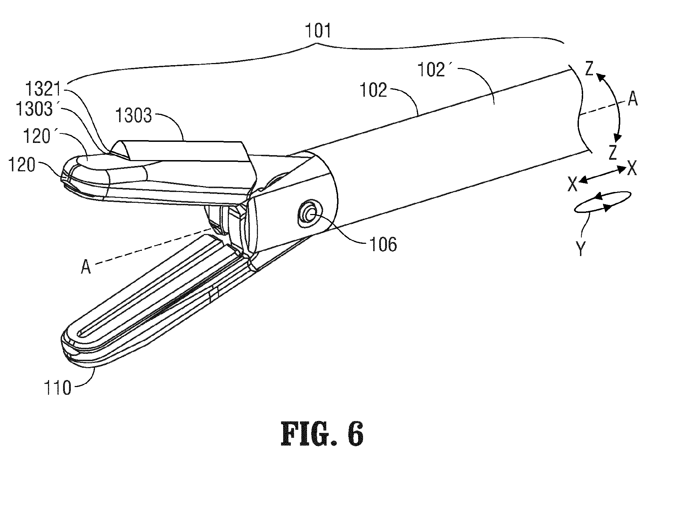

FIG. 6 illustrates one embodiment of the present disclosure wherein a cleaning material 1303 is disposed on the extension member 101, as represented by at least one of the jaw members 110 and 120 of the first surgical instrument 100. More particularly, upper jaw member 120 is illustrated as having cleaning material 1303 disposed on upper surface 120' of the jaw member 120 by means such as an adhesive layer 1321 adhering upper surface 120' to interior surface 1303' of the cleaning material 1303. Again, the cleaning material 1303 may be made from a flexible material such as cloth, rubber or silicone or other suitable material.

The cleaning material 1303 on the jaw member 120 (or on jaw member 110) may be moved in the **X** and/or **Y** and/or **Z** directions described above with respect to FIG. 3. Thus, in a similar manner, the cleaning material 1303 is configured to clean at least a portion of the second surgical instrument 200 when the distance **D1** between the first surgical instrument 100 and the second surgical instrument 200 is reduced sufficiently as described above.

Those skilled in the art will recognize upon reading the foregoing disclosure that FIGS. 1, 1A and 2-6 illustrate a method of cleaning at least a portion of at least second surgical instrument 200 when in proximity to first surgical instrument 100.

The method includes disposing a cleaning material on external surface 102' of extension member 101, e.g., cleaning material 1303 on external surface 120' of jaw member 120 of first surgical instrument 100, as illustrated in FIG. 6, or on an external surface of a shaft of the first surgical instrument, e.g., cleaning material 130 or 1301 or 1302 disposed on external surface 102' of shaft 102 of the first surgical instrument 100 as illustrated in FIGS. 1, 1A and 2-5. The method also includes positioning the first surgical instrument 100 in proximity to second surgical instrument 200 to define distance **D1** therebetween, as illustrated in FIG. 2.

As illustrated in FIG. 3, the method also includes reducing the distance **D1** between the first surgical instrument 100 and the second surgical instrument 200, such as by moving first surgical instrument 100 closer to second surgical instrument 200, or by moving second surgical instrument 200 closer to first surgical instrument 100, or by moving both surgical instruments 100 and 200 at the same time towards each other such that the cleaning material 130 contacts the second surgical instrument 200.

As also illustrated in FIG. 3, the method also includes cleaning at least a portion of the second surgical instrument 200 with the cleaning material 130 when the distance **D1** between the first surgical instrument 100 and the second surgical instrument 200 is reduced as described above.

As illustrated in FIGS. 1, 1A, and 2-5, the step of disposing the cleaning material may include disposing the cleaning material, e.g., cleaning materials 130, 1301 and 1302, on external surface 102' at the distal end 102a of the shaft 102 in proximity to the pair of jaw members 110 and 120.

As illustrated in FIG. 6, the step of disposing the cleaning material may include disposing the cleaning material 1303 on at least one jaw member, e.g., on surface 120' of jaw member 120, as described above.

As illustrated in FIG. 1A, disposing the cleaning material may include, for example, adhering the cleaning material 130 to the shaft 102 via adhesive 132 adhering surface 130' of the cleaning material 130 to external surface 102' of the shaft 102. As illustrated in FIG. 6, disposing the cleaning material may also include adhering the cleaning material 1303 disposed on external or upper surface 120' of the jaw member 120 by means such as adhesive layer 1321 adhering upper surface 120' to interior surface 1303'of the cleaning material 1303.

As illustrated in FIG. 4, the external surface 102' of shaft 102 may define a depression 108 therein and the step of disposing the cleaning material may include disposing the cleaning material 1301 in the depression 108 in the shaft 102.

As illustrated in FIG. 5, the step of disposing the cleaning material may include disposing cleaning material 1302 having a blade-like structure, and the step of cleaning at least a portion of the second surgical instrument 200 when the distance **D1** is reduced may include cleaning at least a portion of the second surgical instrument 200 with the cleaning material 1302 having a blade-like structure, including cleaning at least a portion of the lens 214 of the laparoscopic instrument 200 with the cleaning material 1302 having a blade-like structure.

The step of cleaning may include cleaning at least a portion of the lens 214 of the laparoscopic instrument 200 when the distance **D1** between the first surgical instrument 100 and laparoscopic instrument 200 is reduced sufficiently as described above.

As particularly illustrated in FIG. 3, the step of cleaning may include reciprocally moving the first surgical instrument 100 in the direction of the longitudinal axis **A-A,** as illustrated by arrow **X-X,** or in a zig-zag manner generally in the direction of the longitudinal axis **A-A** (not explicitly shown), such that the cleaning material, e.g. cleaning material 130, 1301, 1302 or 1302, cleans the second surgical instrument 200 when the distance **D1** is reduced sufficiently as described above.

The step of cleaning may also include moving the cleaning material in curved path Y to clean the second surgical instrument 200 when the distance **D1** is reduced sufficiently.

The step of cleaning may also include rotating the cleaning material, e.g. cleaning material 130, 1301, 1302 or 1302, around the longitudinal axis **A-A** such that the cleaning material cleans the second surgical instrument 200 when the distance **D1** is reduced sufficiently.

While several embodiments of the disclosure have been shown in the drawings, it is not intended that the disclosure be limited thereto, as it is intended that the disclosures be as broad in scope as the art will allow and that the specification be read likewise. Therefore, the above description should not be construed as limiting, but merely as exemplifications of particular embodiments.

The invention ,may be described by reference to the following numbered paragraphs:-
1. A first surgical instrument, comprising: a handle assembly defining a proximal end of the first surgical instrument; and an extension member extending distally from the handle assembly and defining a distal end of the first surgical instrument, the extension member defining an external surface, the handle assembly configured to enable a user to move the extension member, wherein the improvement comprises: a cleaning material disposed on the external surface of the extension member at the distal end thereof, the cleaning material configured to clean at least a portion of a second surgical instrument in proximity to the first surgical instrument, the first surgical instrument and the second surgical instrument defining a distance therebetween, the cleaning material configured to clean at least a portion of the second surgical instrument when the distance between the first surgical instrument and the second surgical instrument is reduced.
2. The first surgical instrument according to paragraph 1, wherein the extension member is a shaft having a pair of jaw members at a distal end thereof, the jaw members movable about a pivot from a first position wherein the jaw members are disposed in spaced relation relative to one another to a at least a second position wherein the jaw members are closer to one another for grasping tissue, the shaft and the jaw members each defining external surfaces; and the cleaning material is disposed on the external surface of at least one jaw member or disposed on the external surface of the shaft.
3. The first surgical instrument according to paragraph 2, wherein the cleaning material is disposed on the distal end of the shaft in proximity to the pair of jaw members.
4. The first surgical instrument according to paragraph 1, wherein the second surgical instrument is a laparoscopic instrument including a lens surface, and the cleaning material is configured to clean at least a portion of the lens of the laparoscopic instrument when the distance between the first surgical instrument and the second surgical instrument is reduced.
5. The first surgical instrument according to paragraph 1, wherein the cleaning material is adhered to the extension member via an adhesive adhering a surface of the cleaning material to the external surface of the extension member.
6. The first surgical instrument according to paragraph 2, wherein the cleaning material is adhered to the at least one jaw member or to the shaft via an adhesive adhering a surface of the cleaning material to the external surface of the at least one jaw member or to the external surface of the shaft respectively.
7. The first surgical instrument according to paragraph 1, wherein the extension member defines a depression therein and wherein the cleaning material is disposed in the depression in the extension member.
8. The first surgical instrument according to paragraph 1, wherein the cleaning material includes a blade-like structure, the blade-like structure configured to clean at least a portion of the second surgical instrument when the distance between the first surgical instrument and the second surgical instrument is reduced.
9. The first surgical instrument according to paragraph 8, wherein the blade-like structure is formed from a flexible material.
10. The first surgical instrument according to paragraph 9, wherein the flexible material is made from one of cloth, plastic, rubber or silicone.
11. The first surgical instrument according to paragraph 8, wherein the second surgical instrument is a laparoscopic instrument including a lens surface, the blade-like structure configured to clean at least a portion of the lens of the laparoscopic instrument when the distance between the first surgical instrument and the second surgical instrument is reduced.
12. The first surgical instrument according to paragraph 1, wherein the cleaning material is made from one of cloth, plastic, rubber or silicone.
13. The first surgical instrument according to paragraph 1, wherein the extension member defines a longitudinal axis and the first surgical instrument is configured to reciprocally move in the direction of the longitudinal axis to enable the cleaning material to clean the second surgical instrument when the distance between the first surgical instrument and the second surgical instrument is reduced.
14. The first surgical instrument according to paragraph 1, wherein the first surgical instrument is configured to enable the cleaning material to move in a curved path to clean the second surgical instrument when the distance between the first surgical instrument and the second surgical instrument is reduced.
15. The first surgical instrument according to paragraph 1, wherein the extension member defines a longitudinal axis and the first surgical instrument is configured to rotate the cleaning material around the longitudinal axis to enable the cleaning material to clean the second surgical instrument when the distance between the first surgical instrument and the second surgical instrument is reduced.
16. A method of cleaning at least a portion of a second surgical instrument in proximity to a first surgical instrument, the first surgical instrument comprising a handle assembly and an extension member extending distally from the handle assembly, the method comprising: disposing a cleaning material on an external surface of the extension member; positioning the first surgical instrument in proximity to a second surgical instrument to define a distance therebetween; reducing the distance between the first surgical instrument and the second surgical instrument; and cleaning at least a portion of the second surgical instrument with the cleaning material when the distance between the first surgical instrument and the second surgical instrument is reduced.
17. The method of cleaning according to paragraph 16, wherein the extension member is a shaft having a pair of jaw members at a distal end thereof, the jaw members movable about a pivot from a first position wherein the jaw members are disposed in spaced relation relative to one another to a at least a second position wherein the jaw members are closer to one another for grasping tissue and wherein the step of disposing the cleaning material includes disposing the cleaning material on an external surface of the shaft or on an external surface of at least one jaw member.
18. The method of cleaning according to paragraph 17, wherein the step of disposing the cleaning material includes disposing the cleaning material on the external surface of the shaft at the distal end of the shaft in proximity to the pair of jaw members.
19. The method of cleaning according to paragraph 16, wherein the second surgical instrument is a laparoscopic instrument including a lens surface, the method comprising cleaning at least a portion of the lens of the laparoscopic instrument with the cleaning material when the distance between the first surgical instrument and the second surgical instrument is reduced.
20. The method of cleaning according to paragraph 16, wherein the step of disposing the cleaning material includes adhering the cleaning material to the external surface of the extension member via an adhesive adhering a surface of the cleaning material to the external surface of the extension member.
   1a. A first surgical instrument, comprising:
      a handle assembly defining a proximal end of the first surgical instrument; and
      an extension member extending distally from the handle assembly and defining a distal end of the first surgical instrument,
      the extension member defining an external surface,
      the handle assembly configured to enable a user to move the extension member,
         and
      a cleaning material disposed on the external surface of the extension member at the distal end thereof, the cleaning material configured to clean at least a portion of a second surgical instrument in proximity to the first surgical instrument, the first surgical instrument and the second surgical instrument defining a distance therebetween, the cleaning material configured to clean at least a portion of the second surgical instrument when the distance between the first surgical instrument and the second surgical instrument is reduced.
   2a. The first surgical instrument according to paragraph 1a, wherein the extension member is a shaft having a pair of jaw members at a distal end thereof, the jaw members movable about a pivot from a first position wherein the jaw members are disposed in spaced relation relative to one another to a at least a second position wherein the jaw members are closer to one another for grasping tissue, the shaft and the jaw members each defining external surfaces; and wherein the cleaning material is disposed on the external surface of at least one jaw member or disposed on the external surface of the shaft.
   3a. The first surgical instrument according to paragraph 2a, wherein the cleaning material is disposed on the distal end of the shaft in proximity to the pair of jaw members.
   4a. The first surgical instrument according to any of paragraphs 1a to 3a, wherein the second surgical instrument is a laparoscopic instrument including a lens surface, and the cleaning material is configured to clean at least a portion of the lens of the laparoscopic instrument when the distance between the first surgical instrument and the second surgical instrument is reduced.
   5a. The first surgical instrument according to any of paragraphs 1a to 4a, wherein the cleaning material is adhered to the extension member via an adhesive adhering a surface of the cleaning material to the external surface of the extension member.
   6a. The first surgical instrument according to any of paragraphs 2a to 5a, wherein the cleaning material is adhered to the at least one jaw member or to the shaft via an adhesive adhering a surface of the cleaning material to the external surface of the at least one jaw member or to the external surface of the shaft respectively.
   7a. The first surgical instrument according to any of paragraphs 1a to 6a, wherein the extension member defines a depression therein and wherein the cleaning material is disposed in the depression in the extension member.
   8a. The first surgical instrument according to any of paragraphs 1a to 7a, wherein the cleaning material includes a blade-like structure, the blade-like structure configured to clean at least a portion of the second surgical instrument when the distance between the first surgical instrument and the second surgical instrument is reduced.
   9a. The first surgical instrument according to paragraph 8a, wherein the blade-like structure is formed from a flexible material, preferably, wherein the flexible material is made from one of cloth, plastic, rubber or silicone.
   10a. The first surgical instrument according to paragraph 8a or paragraph 9a, wherein the second surgical instrument is a laparoscopic instrument including a lens surface, the blade-like structure configured to clean at least a portion of the lens of the laparoscopic instrument when the distance between the first surgical instrument and the second surgical instrument is reduced.
   11a. The first surgical instrument according to any of paragraphs 1a to 10a, wherein the cleaning material is made from one of cloth, plastic, rubber or silicone; and/or
      wherein the extension member defines a longitudinal axis and the first surgical instrument is configured to reciprocally move in the direction of the longitudinal axis to enable the cleaning material to clean the second surgical instrument when the distance between the first surgical instrument and the second surgical instrument is reduced; and/or
      wherein the first surgical instrument is configured to enable the cleaning material to move in a curved path to clean the second surgical instrument when the distance between the first surgical instrument and the second surgical instrument is reduced; and/or
      wherein the extension member defines a longitudinal axis and the first surgical instrument is configured to rotate the cleaning material around the longitudinal axis to enable the cleaning material to clean the second surgical instrument when the distance between the first surgical instrument and the second surgical instrument is reduced.
   12a. A method of cleaning at least a portion of a second surgical instrument in proximity to a first surgical instrument,
      the first surgical instrument comprising a handle assembly and an extension member extending distally from the handle assembly, the method comprising:
      disposing a cleaning material on an external surface of the extension member;
      positioning the first surgical instrument in proximity to a second surgical instrument to define a distance therebetween;
      reducing the distance between the first surgical instrument and the second surgical instrument; and
      cleaning at least a portion of the second surgical instrument with the cleaning material when the distance between the first surgical instrument and the second surgical instrument is reduced.
   13a. The method of cleaning according to paragraph 12a, wherein the extension member is a shaft having a pair of jaw members at a distal end thereof, the jaw members movable about a pivot from a first position wherein the jaw members are disposed in spaced relation relative to one another to a at least a second position wherein the jaw members are closer to one another for grasping tissue and wherein the step of disposing the cleaning material includes disposing the cleaning material on an external surface of the shaft or on an external surface of at least one jaw member; and/or
      wherein the step of disposing the cleaning material includes disposing the cleaning material on the external surface of the shaft at the distal end of the shaft in proximity to the pair of jaw members
   14a. The method of cleaning according to any of paragraphs 12a or 13a, wherein the second surgical instrument is a laparoscopic instrument including a lens surface, the method comprising cleaning at least a portion of the lens of the laparoscopic instrument with the cleaning material when the distance between the first surgical instrument and the second surgical instrument is reduced.
   15a. The method of cleaning according to any of paragraphs 12a to 14a, wherein the step of disposing the cleaning material includes adhering the cleaning material to the external surface of the extension member via an adhesive adhering a surface of the cleaning material to the external surface of the extension member.

## Claims

1. A first surgical instrument (100), comprising:
a handle assembly (104) defining a proximal end of the first surgical instrument (100);
an extension member (101) extending distally from the handle assembly (104) and defining a distal end of the first surgical instrument (100),
the extension member (101) defining an external surface (102'), and
the handle assembly (104) configured to enable a user to move the extension member (101);
a cleaning material (1302) disposed on the external surface (102') of the extension member (101) at the distal end (102a) thereof, the cleaning material (1302) configured to clean at least a portion of a second surgical instrument (200) in proximity to the first surgical instrument (100), the first surgical instrument (100) and the second surgical instrument (200) defining a distance therebetween, the cleaning material (1302) configured to clean at least a portion of the second surgical instrument (200) when the distance between the first surgical instrument (100) and the second surgical instrument (200) is reduced,
the cleaning material (1302) including a blade-like structure formed from a flexible material;
**characterized in that**:
the flexible material is made from one of cloth, plastic, rubber or silicone.

2. The first surgical instrument (100) according to claim 1, wherein the cleaning material (1302) is adhered to the extension member (101) via an adhesive adhering a surface of the cleaning material (1302) to the external surface of the extension member (101).

3. The first surgical instrument (100) according to claim 1 or claim 2, wherein the extension member (101) is a shaft (102) having a pair of jaw members (110,120) at a distal end thereof, the jaw members (110, 120) movable about a pivot (106) from a first position wherein the jaw members (110, 120) are disposed in spaced relation relative to one another to at least a second position wherein the jaw members (110, 120) are closer to one another for grasping tissue, the shaft (102) and the jaw members (110, 120) each defining external surfaces; and wherein the cleaning material (1302) is disposed on the external surface of at least one jaw member (110, 120) or disposed on the external surface of the shaft (102).

4. The first surgical instrument (100) according to claim 3, wherein the cleaning material (1302) is adhered to the shaft (102) via an adhesive adhering a surface of the cleaning material (1302) to the external surface of the shaft (102).

5. The first surgical instrument (100) according to claim 3 or claim 4, wherein the cleaning material (1302) is disposed on the distal end of the shaft (102) in proximity to the pair of jaw members (110, 120).

6. The first surgical instrument (100) according to claim 3, wherein the cleaning material (1302) is adhered to the at least one jaw member (110, 120) via an adhesive adhering a surface of the cleaning material (1302) to the external surface of the at least one jaw member (110, 120).

7. The first surgical instrument (100) according to any of the preceding claims, wherein the cleaning material (1302) is configured to clean at least a portion of the lens of a laparoscopic instrument (200) when the distance between the first surgical instrument (100) and the second surgical instrument (200) is reduced.

8. The first surgical instrument (100) according to any of the preceding claims, wherein the extension member (101) defines a longitudinal axis and the first surgical instrument (100) is configured to reciprocally move in the direction of the longitudinal axis to enable the cleaning material (1302) to clean the second surgical instrument (200) when the distance between the first surgical instrument (100) and the second surgical instrument (200) is reduced.

9. The first surgical instrument (100) according to any of the preceding claims, wherein the first surgical instrument (100) is configured to enable the cleaning material (1302) to move in a curved path to clean the second surgical instrument (200) when the distance between the first surgical instrument (100) and the second surgical instrument (200) is reduced.

10. The first surgical instrument (100) according to any of the preceding claims, wherein the extension member (101) defines a longitudinal axis and the first surgical instrument (100) is configured to rotate the cleaning material (1302) around the longitudinal axis to enable the cleaning material (1302) to clean the second surgical instrument (200) when the distance between the first surgical instrument (100) and the second surgical instrument (200) is reduced.
